# EUROPEAN PATENT APPLICATION

(11) **EP 1 552 812 A1**
(43) Date of publication of application: **13.07.2005**
(21) Application number: 02753212.6
(22) Date of filing: 31.07.2002
(51) Int. Cl.: A61K 7/13, A61K 7/135

(54) **EMULSIFIED COMPOSITIONS FOR BLEACHING OR DYEING THE HAIR AND METHODS OF BLEACHING OR DYEING THE HAIR USING THESE EMULSIFIED COMPOSITIONS**

(71) Applicant: Arimino Co., Ltd., Tokyo 161-0033 (JP)
(72) Inventor: MACHIDA, Shoji, ARIMINO CO., Sayama Laboratory, Sayama-shi, Saitama 350-1331 (JP)
(74) Representative: Harding, Charles Thomas
(86) International application number: PCT/JP2002/007778
(87) International publication number: WO 2004/017932

(57) **Abstract**

It becomes possible to provide ammonia-containing hair bleaches or hairdyes which are highly safe and cause less hair damage, skin inflammation and irritation, and have no or reduced irritating odor of ammonia, and a method for bleaching or dyeing hair with the use of the hair bleaches or hairdyes by decreasing an average diameter of emulsified particles of emulsified compositions of ammonia-containing hair bleaches or hairdyes enough to reduce an irritating odor of ammonia.

## Description

### Technical Field

The present invention is intended to provide an emulsified composition for an ammonia-containing hair bleach or hairdye, which is highly safe and causes less hair damage, skin inflammation and irritation, and has no or reduced irritating odor of ammonia, and a method for bleaching or dyeing hair with the use of the emulsified composition.

### Background Art

Emulsified compositions of ammonia-containing oxidative hair bleaches or hairdyes are usually employed in two-pack type, however, there are one-pack and three-pack types other than two-pack type.

### 1. Oxidative hair bleaches and hairdyes of two-pack type

They are comprised of an agent containing alkaline chemicals only or alkaline chemicals and dyes as main components (hereinafter also referred to as the first agent) and an agent containing an oxidant such as hydrogen peroxide as a main component (hereinafter also referred to as the second agent). The two agents are mixed when used for treating hair, and hair bleach or hairdye is conducted.

### 2. Oxidative hairdyes of one-pack type

Hairdyes of one-pack type contain alkaline chemicals and dyes only. They do not contain oxidants, and natural oxidation is occurred in the air.

### 3. Oxidative hair bleaches or hairdyes of three-pack type

Powders of persulfate (oxidant), carbonate (alkaline chemical) or the like are added to hair bleaches or hairdyes of two-pack type. In other words, an agent containing alkaline chemicals only or alkaline chemicals and dyes, an agent containing hydrogen peroxide, and an agent containing persulfate or the like are mixed just before the treatment and used.

Examples of alkaline chemicals contained in the aforementioned agents of one-, two- or three-pack type include ammonia, ethanolamine, carbonate, etc., however, ethanolamine tends to leave its residues and has defects that it causes hair damage and skin irritation, and carbonate has defects that it is not very effective in improving the ability of hydrogen peroxide to bleach or dye hair, and therefore, it cannot make hair color very bright. In contrast, ammonia is highly safe because it is not left on hair or scalp, and does not possibly cause hair damage and skin irritation and further, it has a property to improve the ability of hydrogen peroxide to bleach or dye hair sufficiently, therefore, it is used most commonly. However, ammonia has a serious defect : it emits a characteristic irritating odor when treatments are conducted, and particularly when making hair much brighter, because of the necessity to blend a larger amount of ammonia, a strong irritating odor is emitted and causes discomfort. When a blended amount of ammonia is simply reduced to overcome this defect, bleaching or dyeing performance (hereinafter also referred to as basic performance) is significantly decreased. Consequently, it is an important object to reduce an ammonia odor without decreasing the blended amount of ammonia when possible, while maintaining the basic performance.

As related art for attaining the above-mentioned object, for example, the following means <1> to <4> are suggested (FRAGRANCE JOURNAL 2001-8).
<1> Volatilization of ammonia is suppressed by controlling its form.
   For example, volatilization of ammonia is suppressed by preparing an O/W emulsified substance whose oil phase has a liquid crystal structure of surfactant/water, and confining ammonia into water of a liquid crystal phase of the substance, and both the reduction of ammonia and the maintenance of the basic performance are satisfied.
<2> Volatilization of ammonia is suppressed or ammonia odor is reduced by regulating pH.
   For example, by making a buffer system of ammonia/ammonium bicarbonate or ammonium carbonate, ammonia odor is reduced in comparison to the case of ammonia only, and the basic performance is maintained.
<3> An amount of ammonia is reduced by promoting degradation of hydrogen peroxide significantly with the use of a catalyst, etc.
<4> An alkaline chemical other than ammonia having no irritating odor is penetrated into hair efficiently.

As related art for attaining the above-mentioned object, the followings are exemplified in addition to the aforementioned document.

The followings are compositions aimed at decrease of blended amount of ammonia.

A hair bleach composition of two-pack type comprising the first agent containing monoisopropanolamine and quarternary ammonium salt of fatty acid, and the second agent containing an oxidant, an oxidative hairdye composition of two-pack type comprising the first agent containing monoisopropanolamine, quarternary ammonium salt of fatty acid and an oxidative dye, and the second agent containing an oxidant (Japanese Laid-Open Patent Application No. 2001-122743), and a hairdye composition of two-pack type comprising the first agent containing light isoparaffin and quarternary ammonium salt of fatty acid, and the second agent containing an oxidant (Japanese Laid-Open Patent Application No. 2001-114657).

The followings are compositions aimed at substitution of ammonia.

Ahairdye composition of two-pack type comprising the first agent containing light isoparaffin, and the second agent containing an oxidant (Japanese Laid-Open Patent Application No. 2001-2538), a hair bleach composition of two-pack type comprising the first agent containing monoisopropanolamine and quarternary ammonium salt of fatty acid, and the second agent containing an oxidant (Japanese Laid-Open Patent Application No. 2001-122743), a hairdye composition wherein ammonia water and isopropanolamine are blended with a particular weight ratio (Japanese Laid-Open Patent Application No. 2001-288053), a hairdye composition of two-pack type comprising the first agent containing monoisopropanolamine and at least one kind of alkyl glucosides, and the second agent containing an oxidant (Japanese Laid-Open Patent Application No. 2000-264822), a hairdye composition wherein ammonia water and alkali metal carbonate are blended with a particular weight ratio (Japanese Laid-Open Patent Application No. 2001-328926), and a hairdye composition wherein monoethanolamine and ammonia are blended with a particular weight ratio (Japanese Laid-Open Patent Application No. 10-45547).

The followings are exemplified as a substance which reduces an irritating odor of alkaline chemicals such as ammonia by masking, etc.

A hair cosmetic containing a fragrant component including cis-3-hexenol, a component selected from ammonia, monoethanolamine and aromatic alcohol-based penetration promoter (Japanese Laid-Open Patent Application No. 2002-97122), and a hair treatment composition which comprises an alkaline chemical and a particular aromatic alcohol, and is mixed with an oxidant when used (Japanese Laid-Open Patent Application No. 11-29443).

### Disclosure of the Invention

The present inventor has conducted keen study to attain the above-mentioned object in an emulsified composition for an ammonia-containing oxidative hair bleach or hairdye and a method for bleaching or dyeing hair with the use of the emulsified composition with reduced irritating odor of ammonia . As a result, it has been found that the object, the reduction of irritating odor of ammonia, can be attained in an emulsified composition for a hair bleach or hairdye which contains ammonia as a main component by adopting means for decreasing a diameter of emulsified particles of the emulsified composition, which is the means based on a technical thought completely different from that of the above-mentioned prior art, and thus the present inventor has reached the present invention. In other words, the present inventor has found that it is possible to reduce an irritating odor of ammonia significantly by decreasing an average diameter of emulsified particles of an ammonia-containing emulsified composition for dyeing or bleaching hair (hereinafter referred to as the first agent), which is usually about 50 µm, preferably to 13 µm or less, and particularly to 10 µm or less , and the present inventor has reached the present invention.

In the present invention, the above-mentioned expression "to reduce an irritating odor of ammonia significantly" means that though an irritating odor of the emulsified composition of the first agent mentioned above increases in proportion to the increase of blended amount of ammonia, the decrease of an average diameter of emulsified particles of the composition exhibits a significant effect for reducing the irritating odor of ammonia in comparison with conventionally known ammonia-containing emulsified compositions when the same amount of ammonia is blended. Therefore, it becomes possible to reduce an irritating odor of ammonia in comparison with conventionally known ammonia-containing emulsified compositions when the same amount of ammonia is blended, and to use an amount of ammonia required to achieve the basic performance sufficiently while reducing its irritating odor.

Further, in the present invention, the reduction of an irritating odor of ammonia means that an irritating odor of ammonia is reduced to the degree that persons who treat hair or persons who have their hair treated do not smell an irritating odor of ammonia or slightly smell an ammonia odor when the first agent of the present invention is used for dyeing or bleaching hair.

The present invention will be described more specifically with embodiments of the present invention, but the present invention is not limited to the following embodiments.

### A. The first agent

As the first agent mentioned above used in the present invention, commercially available emulsified compositions of ammonia-containing hair bleaches or hairdyes are exemplified, and 28% ammonia water of about 3 to 15 wt%, usually about 6 to 8 wt%, is used in the emulsified compositions. Further, the viscosity of the first agent and an oxidant mixed to the first agent is preferably adjusted to be about 10,000 to 60,000 cps.

The first agents of hairdyes and hair bleaches of the present invention comprise, for example, at least the following (1) to (3).
(1) Ammonia
(2) Surfactant
(3) Other components of the emulsified composition (oleum, silicon, water, etc.)

### (1) Ammonia

In the first agent of the present invention, the effect for reducing an irritating odor of ammonia increases in proportion to the decrease of an average diameter of emulsified particles as mentioned above. In addition, in order to reduce an irritating odor of ammonia, it is preferable to decrease an average diameter of emulsified particles in proportion to the increase in an amount of ammonia blended into the first agent. Accordingly, there arises the effect for reducing an irritating odor of ammonia sufficiently when an average diameter of emulsified particles is decreased to about 13 µm, preferably to about 13 µm to 5 nm, as aforementioned. The effect for reducing an irritating odor of ammonia further increases when an average diameter of emulsified particles is decreased preferably to 1 µm or less, for example, to about 1 µm to 5 nm, more preferably to nm order. Therefore, the present invention makes it possible to solve both problems of an irritating odor of ammonia and securing of the basic performance such as bleaching or dyeing performance, compatibly, without adopting means for using substitutes of ammonia, adding a masking agent for an irritating odor of ammonia, or adding a promoter of degradation of hydrogen peroxide, as in the cases of prior art mentioned above.

The above-mentioned average diameter of emulsified particles can be controlled by properly modifying at least one or two or more of requirements, for example, blended composition, blended order, means for stirring or degree of stirring of components which comprise the first agent.

The above-mentioned average diameter of emulsified particles was measured with a laser diffraction particle size analyzer SALD-2000 (Shimadzu) and a submicron particle size analyzer N4S (Coulter) used in the following examples.

The content of ammonia used in the first agent of the present invention is 1 to 30 wt% of the emulsified composition in case where commercially available 28% ammonia aqueous solution is used. When the content of ammonia is less than 1wt%, a sufficient effect for bleaching or dyeing hair is not obtained, and when the content of ammonia exceeds 30 wt% , possibility of skin irritation and strong irritating odor arise, and severer discomfort is caused.

In addition to ammonia, other alkaline chemicals may be added to the first agent mentioned above. Examples of such alkaline chemicals include monoethanolamine, diethanolamine, triethanolamine, morpholine, ammonium carbonate, ammonium hydrogen carbonate, sodium carbonate, sodium hydrogen carbonate, guanidine hydrogen carbonate. One or more kinds of these alkaline chemicals can be used together with ammonia within a range of 0.1 to 10 wt% of the emulsified composition.

### (2) Surfactant

As a surfactant used as an emulsifier of the composition of the first agent, a nonionic surfactant is particularly preferable. It is possible to blend one kind of, or a mixture of two or more kinds of nonionic surfactants into the composition. The blended amount is not particularly limited as long as it is a sufficient amount to obtain fine emulsified particles, however, the blended amount is 0.1 to 50 wt%, preferably 0.1 to 30 wt%, more preferably 0.1 to 10 wt% of the emulsified composition.

As to the amount of the nonionic surfactant used, when the amount is less than 0.1 wt%, sufficient emulsifying ability is not obtained, and it is not economical and may cause a negative influence on dyeing property to blend the amount of the nonionic surfactant exceeding 50 wt%.

As a surfactant, besides the nonionic surfactant, one or more kinds of ionic surfactants selected from anionic, cationic, and amphoteric surfactants can be blended alone, or together with a nonionic surfactant. However, certain kinds and/or blended amounts of anionic, cationic, and amphoteric surfactants may cause a negative influence on the effect for reducing an irritating odor of ammonia, therefore, anionic, cationic, and amphoteric surfactants can be used to the extent that they do not cause a negative influence on the effect for reducing an irritating odor of ammonia, or that the effect for reducing an irritating odor of ammonia, which is the object of the present invention, can be attained. For example, in case where an ionic surfactant is blended alone or together with a nonionic surfactant, a blended amount of the ionic surfactant is preferably 2 wt% or less, although it depends on the kinds of ionic surfactants used. In particular, blending of one or more kinds of ionic surfactants results in better emulsification, nicer touch of hair and easier removal of color of hairdyes attached to scalps when treatments are conducted. In that case, the blended amount of the ionic surfactant mentioned above is 2 wt% or less, preferably 1 wt% or less of the first agent. When the blended amount is 2% or more, there would be difficulty in reducing an irritating odor of ammonia when it is mixed with the second agent as mentioned above, even if diameters of emulsified particles are decreased.

The nonionic surfactants used in the present invention are not particularly limited and known nonionic surfactants conventionally used for hair bleaches and hairdyes can be widely used.

Examples of the nonionic surfactants include polyoxyethylene alkyl ethers such as polyoxyethylene isostearyl ether, polyoxyethylene isocetyl ether, polyoxyethylene oleyl ether, polyoxyethylene oleylcetyl ether, polyoxyethylene stearyl ether, polyoxyethylene cetyl ether, polyoxyethylene cetostearyl ether, polyoxyethylene butyl ether, polyoxyethylene behenyl ether, polyoxyethylene myristyl ether, polyoxyethylene lauryl ether, polyoxyethylene tridecyl ether, polyoxyethylene hexyldecylether, polyoxyethylene octyldodecyl ether, polyoxyethylene decylpentadecyl ether, polyoxyethylene decyltetradecyl ether; polyoxy alkylphenyl ethers such as polyoxyethylene octylphenyl ether, polyoxyethylene dinonylphenyl ether, polyoxyethylene nonylphenyl ether; polyoxyethylene polyoxypropylene alkyl ethers such as polyoxyethylene polyoxypropylene stearyl ether, polyoxyethylene polyoxypropylene cetyl ether, polyoxyethylene polyoxypropylene decyltetradecyl ether, polyoxyethylene polyoxypropylene butyl ether,polyoxyethylene polyoxypropylene lauryl ether; polyvalent alcohol fatty acid esters such as propylene glycol monostearate; glycerin fatty acid esters such as glyceryl monomyristate; polyglycerin fatty acid esters such as decaglyceryl monolaurate; sorbitan fatty acid esters such as sorbitan monopalmitate; polyoxyethylene glycerin fatty acid esters such as polyoxyethylene glyceril monostearate; polyoxyethylene sorbitan fatty acid esters such as polyoxyethylene sorbitan trioleate; polyoxyethylene sorbit fatty acid esters such as polyoxyethylene sorbit tetraoleate; polyoxyethylene glycol fatty acid esters such as polyoxyethylene glycol monostearate; alkyl alkanol amides such as coconut fatty acid diethanol amide; polyoxyethylene hydrogenated castor oil; polyoxyethylene lanolin; polyoxyethylene cholesterol; polyoxyethylene phytosterol; polyoxyethylene cholestanol; polyoxyethylene phytostanol.

The ionic surfactants used in the present invention are not particularly limited either, and anionic, cationic, and amphoteric surfactants conventionally used for dyeing or bleaching hair can be properly used.

Examples of anionic surfactants include fatty acid soap, alkyl ether carboxylate, fatty acid amide ether carboxylate, acyl lactate, N-acyl glutamate, N-acyl alanate, N-acyl sarcosinate, N-acyl-ω-amino-acid salt, alkane sulfonate, α-olefin sulfonate, α-sulfofatty acid methyl ester salt, acyl isethionate, alkyl glycidyl ether sulfonate, alkyl sulfosuccinate, alkyl sulfoacetate, alkyl benzene sulfonate, alkyl naphthalene sulfonate, N-acyl methyl taurate, formalin-condensed sulfonate, alkyl sulfate, polyoxyethylene alkyl ether sulfate, alkyl aryl ether sulfate, fatty acid alkanol amide sulfate, fatty acid monoglyceride sulfate, alkylphosphate, polyoxyethylene alkyl ether phosphate, alkyl aryl ether phosphate, and fatty acid amide ether phosphate.

Examples of cationic surfactants include quaternary ammonium salts such as stearyl chloride trimethyl ammonium, cetyl chloride trimethyl ammonium, alkyl chloride trimethyl ammonium, distearyl chloride dimethyl ammonium; fatty acid amide amine salt; alkyl trialkylene glycol ammonium salt; benzalkonium salt; benzethonium salt; pyridium salt; imidazolinium salt.

Examples of amphoteric surfactants include those of glycine type, aminopropionic acid type, alkyl betaine type, alkyl amide betaine type, imidazoline type, amine oxide type, sulfobetaine type, sulfonic acid type, sulfuric acid type, and phosphoric acid type.

As the forms of emulsified substances of the first agent mentioned above, O/W emulsions or W/O emulsions are exemplified, however, combined emulsions in which O/W emulsions or W/O emulsions are dispersed in water are more preferable.

### (3) Other components of the emulsified composition (oleum, silicon, water, etc.)

An oleum can be added to the first agent of the present invention. Examples of such oleum include higher alcohols such as behenyl alcohol, stearyl alcohol, cetostearyl alcohol, cetanol, lauryl alcohol; oils such as avocado oil, olive oil, macadamia nut oil, castor oil; hydrocarbons such as liquid paraffin, squalene, microcrystalline wax; higher fatty acids such as lauric acid, myristic acid, stearic acid; esters such as isopropyl myristate, isostearyl isostearate, myristyl lactate.

It is possible to blend one kind of, or a mixture of two or more kinds of the olea mentioned above, and blended amount is 0.1 to 30 wt%, preferably 0.5 to 20 wt% of the emulsified composition.

An oxidization dye is blended into compositions of the first agent for dyeing hair of the present invention. Specific examples of the dye include phenylenediamines, aminophenols, toluilenediamines, aminonitrophenols, diphenylamines, diaminodiphenylamines, N-phenylphenylenediamines, aminopyridines and salts thereof. In addition to them, couplers such as resorcin, pyrogallol, catechol, metaminophenol, metaphenylenediamine, can be blended. Further, substances listed in "The Japanese Standards for Quasi-Drug Ingredients" (The Yakuji Nippo Limited, issued June, 1991) are also specific examples of the oxidization dye and the coupler, and can be used. It is possible to use one kind of, or a combination of two or more kinds of these oxidization dyes and couplers respectively, and blended amount is not particularly limited but is preferably 0.01 to 20 wt%, particularly preferably 0.1 to 10 wt% of the emulsified composition.

Besides the components mentioned above, other optional components usually used in the cosmetic field can be blended into the first agent of the present invention to the extent that they do not interfere with the effect of the present invention. Examples of these optional components include moisturizers such as glycerin, propyleneglycol, polyethyleneglycol; stabilizers such as sulfite, ascorbic acid, thioglycollate, cysteine, edetate; conditioners such as cationized cellulose, cationized guar gum, cationized polymer, cationized resin; silicones such as methyl polysiloxane, polyether modified silicone, amino modifiedsilicone; hair protectants such as protein, polypeptide, amino acid; thickeners such as carboxyethyl cellulose, xanthan gum; UV-radiation absorbents; preservatives; pearl brighteners; penetrants; humectants; pilatory agents; colorants; flavors.

The forms of the first agent of the present invention are not particularly limited, and as long as the agent is an emulsified composition, various forms such as cream, emulsion, aerosol foam, mist, spray, can be employed.

The first agent of the present invention can be produced by an ordinary method, however, regarding the mixing and stirring of each component, each component is gradually added and the resulting mixture is stirred enough to be a minute emulsified substance.

### B. The second agent

The first agent of the present invention is mixed with the second agent containing an oxidant such as hydrogen peroxide, and used to treat hair. This mixing of the first and the second agents is usually conducted just before the treatment.

Examples of the oxidant used as the second agent include hydrogen peroxide and urea peroxide, and the concentration in use depends on the brightness of hair required. For example, hydrogen peroxide is used usually at a concentration of about 0.5 to 15 wt%, more preferably, about 0.5 to 12 wt%. Further, stabilizers such as phenacetin and edetate, surfactants, oils and fats, hydrocarbons, higher alcohols, higher fatty acids, esters, conditioners, hair protectants, thickeners, acids, pH regulators, flavors, colorants, etc., can be blended properly into the second agent, in addition to oxidants.

The forms of the second agent are not particularly limited, and for example, forms such as liquid, emulsion, cream, gel, aerosol foam, mist, spray, can be employed, but it is preferable that the form of the second agent is the same as that of the first agent.

In case where the second agent is used as an emulsified composition in a same manner as in the case where the first agent is used, a nonionic surfactant is preferable as a surfactant, and from the viewpoint of the reduction of an irritating odor of ammonia, as in the case of the first agent, the effect becomes prominent in proportion to the decrease of an average diameter of emulsified particles. In addition, it is preferable that the first and the second agents mentioned above have a same degree of physical properties, in particular, a same degree of viscosity. It is particularly preferable that the first and the second agents have the same form, and a same degree of viscosity wherein dripping is prevented on hair.

The use of the first and the second agents having a same degree of viscosity provides not only easier mixing of these agents, but also complete solution to problems such as dripping of emulsified compositions or difficulty in treatment due to the hardness of the compositions, because the viscosity is constant even though these agents are mixed with an optional combination and/or an optional ratio. Therefore, persons who treat hair can mix the first and the second agents easily at a ratio of their own choice, and can change the brightness and color tone of hair freely. For example, by increasing the mixing ratio of the second agent to enhance oxidizing ability, stronger bleach is conducted. In case of hair bleach, original hair color is removed more strongly, and in case of hairdye, it becomes possible to dye hair a brighter color tone.

As the first and the second agents having substantially the same viscosity which prevents dripping on hair, a creamy agent of about 10,000 to 60,000 cps is preferable, and of about 20 , 000 to 45, 000 cps is more preferable. In the present invention, "the viscosities of the first and the second agents are substantially the same" does not mean that the viscosities of both agents are exactly the same. The viscosities of the first and the second agents are regarded as substantially the same if persons who treat hair can mix the first and the second agents easily at a ratio of their own choice, and can change the brightness and color tone of hair freely, as mentioned above, even though there is a difference in the viscosities of the first and the second agents.

In the aforementioned embodiments of the present invention, hair bleaches and hairdyes are described with reference to two-pack type which uses the first and the second agents mentioned above, however, even in case of one- or three-pack type, in case where an ammonia-containing emulsified composition is used, an ammonia odor can be reduced by decreasing diameters of emulsified particles of the emulsified composition.

Also in three-pack type, in particular, when an agent containing an oxidant is an emulsified composition, the effect for reducing an ammonia odor can be increased in proportion to the decrease of diameters of emulsified particles of the agent.

It is possible to bleach or dye hair with the use of the ammonia-containing emulsified composition mentioned above. In one-pack type, an amount of the composition used for one treatment is put into a cup and used for the treatment by an ordinary method. In two- and three-pack types, equal amounts of the first agent containing ammonia used for one treatment and the second agent containing an oxidant are put into a cup, and a powder composition, the third agent, is added in three-pack type, and the mixture is stirred with a brush, and used for the treatment by an ordinary method.

As for a treating method which further increases the effect of the present invention, in case of two- and three-pack types, it is preferable to make the first agent containing ammonia and the second agent containing an oxidant have a same degree of physical properties, particularly viscosity, and to use them for treatments. As aforementioned, the use of the first and the second agents having a same degree of viscosity can provide not only an easy and uniform mixing of these agents, but also an optional setting of a mixing ratio of the first and the second agent at the time of treatment, and as a result, an optional color tone can be obtained. In this case, it is preferable that the viscosity of the emulsified composition is 10,000 to 60,000 cps.

### Best Mode of Carrying Out the Invention

The present invention will be described more specifically with best modes of carrying out the invention, but the present invention is not limited to these modes. In the followings, average diameters of emulsified particles of the first agents prepared in examples were measured, the first agents were mixed with the second agents in reference examples, and evaluation was conducted according to the evaluation method mentioned below. The results are shown in Table 3.

### Evaluation method

### (1) Measurement of average diameters of emulsified particles

Emulsified hairdye compositions were properly diluted with purified water, and the average particle diameters of the diluted solutions were measured with a laser diffraction particle size analyzer SALD-2000 (Shimadzu) and a submicron particle size analyzer N4S (Coulter).

### (2) Ammonia odor test

10 g of emulsified hairdye composition and 10 g of the second agent were put into a cup, stirred with a brush, and used as a sample. In each example, 20 monitors were divided into 5 groups of 4 persons, and fresh samples were prepared for each group. Each monitor further stirred the sample with a brush one or two times on evaluation, and smelled the sample. Sensory evaluation was conducted according to the following standards.
ⓞ: indicates that almost no irritating odor is sensed.
○: indicates that it has a faint irritating odor.
Δ: indicates that it has a considerable irritating odor.
×: indicates that it has an overpowering irritating odor.

### (3) Hairdye test

Emulsified hairdye composition and the second agent were put into a cup at a ratio of 1:1, and the mixture was stirred with a brush and applied to a tuft of human black hair. The tuft of human black hair was left for 30 minutes at room temperature, and then shampooed, rinsed and dried. After hairdye was completed, each tuft of hair was compared by macroscopic evaluation.

### Examples 1 ~ 2

In each example, blended components were arranged to be 800 g in total according to the blended amounts described in Table 1 shown below, a 1-liter glass beaker was used, and the blended components were stirred manually with two glass rods 8 mm in diameter.

Specifically, components other than paraphenylenediamine, resorcin, sodium sulfite, 28% ammonia water, purified water, were melted by heat at 75°C (oily components). Paraphenylenediamine, resorcin, sulfite, purified water (aqueous components) were melted by heat at 75°C while being stirred moderately, and this mixture was gradually added to the oily components, which was being stirred as fast as possible (about 1 to 2 rotation/sec). The resulting mixture was stirred in a similar manner and cooling of the mixture was started. The mixture was continued to be stirred as fast as possible (the speed was reduced to about 1 rotation/sec) even when the mixture was cooled to about 50° C and the viscosity started to increase, and then, the mixture was cooled to 30°C. Next, 28% ammonia water was added to the mixture, and the stirring was continued to the resulting mixture in a similar manner, thus a creamy emulsified composition (the first agent) was arranged. It took about 15 minutes to complete the arrangement of the creamy emulsified composition (the first agent) from when the mixing of the oily components and the aqueous components was started.

Example 3 was conducted in a manner similar to that of Examples 1 - 2, however, the speed of stirring for mixing the oily components and the aqueous components was consistently slower (0.5 to 1 rotation/sec).

The obtained average diameters of emulsified particles of Examples 1 - 3 are indicated in Table 3 shown below.

**Table 1**

| Blended components | Example 1 | Example 2 | Example 3 |
|---|---|---|---|
| Cetanol | 4.0 | 4.0 | 6.0 |
| Liquid paraffin | 8.0 | 8.0 | 6.0 |
| Polyoxyethylene stearyl ether | 6.0 | 5.0 | 6.0 |
| Stearyl chloride trimethyl ammonium (70%) | - | 1.0 | - |
| Polyethylene glycol | 5.0 | 5.0 | 5.0 |
| Paraphenylenediamine | 0.5 | 0.5 | 0.5 |
| Resorcin | 0.5 | 0.5 | 0.5 |
| Sodium sulfite | 0.5 | 0.5 | 0.5 |
| 28% ammonia water | 8.0 | 8.0 | 8.0 |
| Purified water | Amount that makes 100 wt% in total | | |

The values in Table 1 shown above indicate wt%.

### Reference Example 1 (Method for preparing the second agent)

An aqueous solution of the second agent was prepared by adding purified water to 17 wt% of 35% hydrogen peroxide solution to make 100 wt% of the resulting solution.

### Reference Examples 2 ~ 3 (Method for preparing the second agent)

According to the blended amounts described in Table 2 shown below, components other than 35% hydrogen peroxide solution and purified water were melted by heat at 75° C, and purified water, heated to 75°C, was gradually added to the mixture of the components while being stirred, and the stirring was continued to the resulting mixture. When the mixture was cooled to 30° C, another 35% hydrogen peroxide solution was added and a creamy emulsified composition was obtained. The average particle diameter of the emulsified composition obtained in Reference Example 2 was 202 nm, and that of the emulsified composition obtained in Reference Example 3 was 4.086 µm.

**Table 2**

| | Reference Example 2 | Reference Example 3 |
|---|---|---|
| Cetanol | 5.0 | 5.0 |
| Liquid paraffin | 10.0 | 5.0 |
| Polyoxyethylene stearyl ether | 6.0 | 4.0 |
| Polyethylene glycol | 5.0 | 5.0 |
| 35% hydrogen peroxide solution | 17.0 | 17.0 |
| Purified water | Amount that makes 100 wt% in total | |

The values in Table 2 shown above indicate wt%.

**Table 3**

| | Average diameters of emulsified particles (the first agents) | Ammonia odor test | | | |
|---|---|---|---|---|---|
| | | ⓞ | ○ | Δ | × |
| Example 1 | 392 nm | 17 | 3 | | |
| Example 1A | 392 nm | 20 | | | |
| Example 1B | 392 nm | 18 | 2 | | |
| Example 2 | 288 nm | 11 | 9 | | |
| Example 3 | 7.865 µm | 5 | 14 | 1 | |

The values in the ammonia odor test in Table 3 shown above indicate the number of test subjects. Further, in the ammonia odor test, the second agent of Reference Example 1 was used in Examples 1 ~ 3, and the second agent of Reference Example 2 was used in Example 1A, and the second agent of Reference Example 3 was used in Example 1B. As a result of the hairdye test, tufts of hair were dyed in the same color tone in all Examples in Table 3.

### Example 4 (Method for dyeing hair)

60 g of the emulsified composition obtained in Example 1 was put into a cup, and 60 g of the emulsified composition obtained in Reference Example 2 was added to the aforementioned composition. The resulting mixture was stirred with a brush, and applied to dye black hair brown in accordance with an ordinary method. In the process of hairdye, there was little ammonia odor, and as a result of the hairdye test, tufts of hair were dyed in the same color tone as in other Examples.

### Industrial Applicability

The present invention makes it possible to provide an ammonia-containing oxidative hair bleach or hairdye, wherein an irritating odor of ammonia can be reduced to the extent nearly imperceptible when an emulsified composition for an ammonia-containing hair bleach or hairdye and an agent containing an oxidant such as hydrogen peroxide are mixed and used for treating hair, and a method for bleaching or dyeing hair with the use of the hair bleach or hairdye.

## Claims

1. An emulsified composition for an ammonia-containing hair bleach or hairdye, wherein an average diameter of emulsified particles of said composition is small enough to reduce an irritating odor of ammonia.

2. The emulsified composition for an ammonia-containing hair bleach or hairdye according to claim 1, wherein the average diameter of emulsified particles of said composition is 10 µm or less.

3. The emulsified composition for an ammonia-containing hair bleach or hairdye according to claim 1 or 2, comprising a nonionic surfactant of 0.1 to 50 wt%.

4. The emulsified composition for an ammonia-containing hair bleach or hairdye according to claim 3, comprising one or more kinds of ionic surfactants of 2 wt% or less, selected from an anionic surfactant, a cationic surfactant, and an amphoteric surfactant.

5. A hair bleach orhairdye which comprises at least the emulsified composition for a hair bleach or hairdye according to claim 1, 2, 3, or 4, and an oxidant.

6. The hair bleach or hairdye according to claim 5, wherein the emulsified composition for an ammonia-containing hair bleach or hairdye and a composition containing the oxidant has a same viscosity.

7. The hair bleach or hairdye according to claim 5 or 6, wherein the viscosities of the emulsified composition for an ammonia-containing hair bleach or hairdye and the composition containing the oxidant are 10,000 to 60,000 cps.

8. A method for bleaching or dyeing hair which uses the hair bleach or hairdye according to claim 1, 2, 3, 4, 5, 6 or 7.
